# DEMANDE DE BREVET EUROPEEN

(11) **EP 1 994 900 A1**
(43) Date de publication de la demande: **26.11.2008**
(21) Numéro de dépôt: 07010112.6
(22) Date de dépôt: 22.05.2007
(51) Int. Cl.: A61B 17/70

(54) **Implant vertébral inter-épineux**

(71) Demandeur: Flexismed SA, 1204 Genève (CH)
(72) Inventeur: Ben-Mokhtar, Mourad, 1207 Genève (CH); Fuentes, Jean Marc, 34790 Montpellier (FR)
(74) Mandataire: Micheli & Cie SA

(57) **Abrégé**

Un implant vertébral inter-épineux comprenant des premier et second supports (2, 3) définissant dans leur partie centrale des organes respectifs (7, 8) de fixation à des apophyses épineuses de deux vertèbres respectives, ces organes de fixation (7, 8) étant situés dans un même plan sagittal (P), des premier et second organes élastiquement compressibles (4) dans la direction du rachis, ces organes élastiquement compressibles (4) étant disposés entre les premier et second supports (2, 3) et de part et d'autre dudit plan sagittal (P), est caractérisé en ce qu'il comprend en outre des premier et second organes rigides de guidage (10) autour desquels sont disposés respectivement les premier et second organes élastiquement compressibles (4), chacun de ces organes de guidage (10) étant connecté à ses deux extrémités aux supports (2, 3) et comportant à au moins l'une de ses extrémités une butée (13) conçue pour coopérer avec une butée (14) du support correspondant (3) pour limiter l'écartement des supports (2, 3) suivant la direction du rachis.

## Description

La présente invention concerne un implant vertébral inter-épineux, c'est-à-dire un implant destiné à être inséré entre les apophyses épineuses de deux vertèbres adjacentes pour servir de stabilisateur et soulager notamment le disque intervertébral.

On connaît par la demande de brevet US 2002/0143331, figure 9, un implant vertébral inter-épineux comprenant des premier et second supports définissant des organes de fixation à des apophyses épineuses respectives adjacentes et entre lesquels est disposé un organe élastique de forme annulaire et coaxial avec les supports. L'organe élastique est disposé entre le fond d'une cavité annulaire du premier support et un organe mâle annulaire du second support qui est guidé axialement dans cette cavité. La paroi périphérique du premier support comprend une saillie annulaire interne qui peut coopérer avec un épaulement annulaire de l'organe mâle du second support pour limiter l'écartement des supports. La face d'extrémité de la paroi périphérique du premier support constitue également une butée qui peut coopérer avec un bord du second support pour limiter la compression de l'organe élastique.

Cet implant selon le document US 2002/0143331 présente plusieurs inconvénients. En premier lieu, on peut noter que cet implant et son organe élastique ont une forme circulaire. Pour obtenir une certaine capacité d'amortissement, l'organe élastique doit avoir des dimensions suffisantes. Or si l'on augmente le diamètre de l'implant, son encombrement dans la direction antéro-postérieure sera trop grand et nécessitera de délabrer dans une trop grande mesure les tissus vertébraux pour la mise en place de l'implant. D'autre part, la présence de l'organe mâle annulaire du second support et de la paroi périphérique du premier support limite de façon importante l'espace disponible pour l'organe élastique. Un autre inconvénient est que les butées qui limitent l'écartement des supports apparaissent sous-dimensionnées pour pouvoir véritablement résister à des charges physiologiques.

La demande de brevet FR 2 774 581, figure 4, décrit un implant vertébral inter-épineux comprenant les caractéristiques mentionnées dans le préambule de la revendication 1 annexée, à savoir :
- des premier et second supports définissant dans leur partie centrale des organes respectifs de fixation à des apophyses épineuses de deux vertèbres respectives, ces organes de fixation étant situés dans un même plan sagittal, et
- des premier et second organes élastiquement compressibles dans la direction du rachis, ces organes élastiquement compressibles étant disposés entre les premier et second supports et de part et d'autre dudit plan sagittal.

Les organes élastiquement compressibles sont sous la forme de soufflets définissant des enceintes étanches remplies d'un fluide et d'un noyau en un matériau viscoélastique. Cet implant selon le document FR 2 774 581 semble présenter une plus grande capacité d'amortissement que celui décrit dans le document US 2002/0143331. Toutefois, cet implant apparaît difficilement réalisable car les extrémités des soufflets doivent être solidement fixées aux supports pour maintenir les enceintes étanches et éviter que les supports se détachent l'un de l'autre. Or le document FR 2 774 581 n'explique pas comment fixer les soufflets aux supports. Ce problème de fixation des soufflets aux supports est d'autant plus critique que l'implant ne comporte pas de butées pour limiter l'écartement des supports ni pour limiter la compression des soufflets. Il convient de noter de plus que les noyaux en matériau viscoélastique ne sont pas vraiment guidés et sont donc susceptibles de se déplacer latéralement ce qui peut déséquilibrer l'implant et perturber le fonctionnement des soufflets.

La présente invention vise à remédier aux inconvénients précités de l'état de la technique et propose à cet effet un implant vertébral inter-épineux comprenant les caractéristiques mentionnées plus haut en rapport avec le document FR 2 774 581 et caractérisé en ce qu'il comprend en outre des premier et second organes rigides de guidage autour desquels sont disposés respectivement les premier et second organes élastiquement compressibles, chacun de ces organes de guidage étant connecté à ses deux extrémités aux supports et comportant à au moins l'une de ses extrémités une butée conçue pour coopérer avec une butée du support correspondant pour limiter l'écartement des supports suivant la direction du rachis.

Ainsi, les organes rigides assurent à la fois une fonction de guidage et une fonction de butée. Cet agencement permet de garantir que les organes élastiques resteront bien en place dans l'implant et de conférer une grande robustesse aux butées. L'implant selon l'invention peut présenter un faible encombrement dans la direction antéro-postérieure, ce qui permet notamment de conserver une bonne partie des ligaments naturels. L'encombrement de l'implant dans la direction latérale est peu gênant car les tissus correspondants sont surtout constitués par du muscle.

Des modes de réalisation particuliers de l'invention sont définis dans les revendications dépendantes annexées 2 à 12.

D'autres caractéristiques et avantages de la présente invention apparaîtront à la lecture de la description détaillée suivante de plusieurs modes de réalisation de l'invention faite en référence aux dessins annexés dans lesquels :
- les figures 1 et 2 sont respectivement une vue en perspective et une vue en coupe frontale d'un implant vertébral inter-épineux selon un premier mode de réalisation ;
- les figures 3 et 4 sont des vues en coupe frontale d'un implant vertébral inter-épineux selon un second mode de réalisation, respectivement dans une position droite et dans une position inclinée ;
- la figure 5 est une vue plane frontale montrant un implant selon l'invention en place entre deux apophyses épineuses ;
- les figures 6 et 7 sont respectivement une vue en perspective et une vue de profil d'une pièce d'arrêt utilisée pour arrêter un ligament servant au maintien de l'implant selon l'invention entre deux apophyses épineuses ; et
- la figure 8 est une vue en perspective de la pièce d'arrêt dans un état intermédiaire non plié.

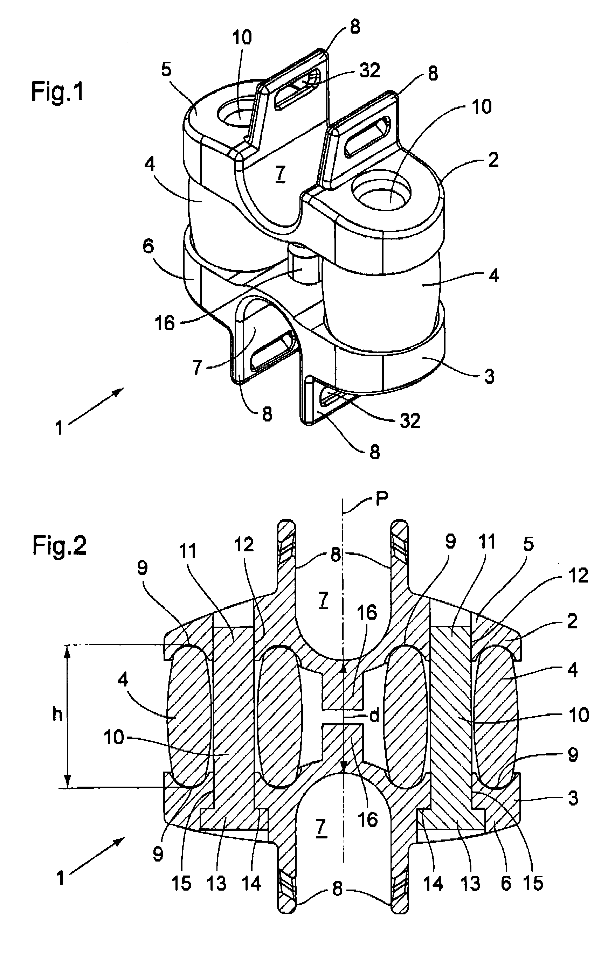

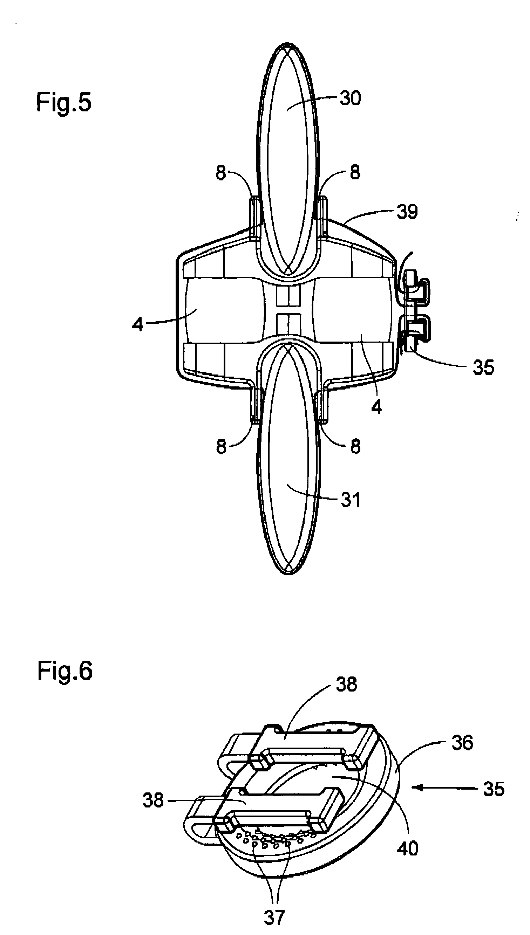

En référence aux figures 1 et 2, un implant vertébral inter-épineux 1 selon un premier mode de réalisation de l'invention comprend un support supérieur 2, un support inférieur 3 et deux organes élastiques 4 interposés entre les supports 2, 3. Chaque support 2, 3 comprend une platine 5, 6 allongée dans la direction latérale et dans la partie centrale de laquelle est prévu un évidement 7 en forme de U s'étendant dans la direction antéro-postérieure. Les parois latérales de l'évidement 7 sont prolongées par une paire d'ailettes 8 qui fait saillie sur la surface extérieure de la platine 5, 6. La paire d'ailettes 8 forme avec l'évidement 7 un organe de fixation à une apophyse épineuse. Plus précisément, l'évidement 7 et l'espace entre les ailettes 8 sont destinés à recevoir une apophyse épineuse, le maintien de cette dernière dans l'organe 7, 8 étant assuré par exemple par un ou des ligaments synthétiques comme cela sera expliqué plus loin en relation avec la figure 5. Les organes de fixation respectifs 7, 8 des supports 2, 3 sont situés dans un même plan sagittal P, qui constitue un plan de symétrie pour l'implant 1. Chaque support 2, 3, avec sa platine 5, 6 et ses ailettes 8, est typiquement une pièce monobloc réalisée en un matériau biocompatible, par exemple métallique, de plus grande dureté que cette des organes élastiques 4.

Les organes élastiques 4 sont alignés dans la direction latérale et sont situés de part et d'autre du plan sagittal de symétrie P. Les organes élastiques 4 sont élastiquement compressibles dans la direction du rachis (direction verticale P sur la figure 2) et sont typiquement réalisés en une matière viscoélastique telle que le silicone. Les organes élastiques 4 ont une forme générale cylindrique annulaire et leurs extrémités reposent, sans y être fixées par un autre moyen, dans des creusures annulaires 9 pratiquées dans les faces intérieures en regard des platines 5, 6. Chaque organe élastique 4 entoure une tige de guidage rigide 10, typiquement métallique, qui s'étend parallèlement au plan P, dans la direction du rachis, et qui est connectée à ses deux extrémités aux platines 5, 6 des supports 2, 3, respectivement. Chaque tige de guidage 10 est plus précisément sous la forme d'une vis dont l'extrémité filetée 11 est vissée dans un alésage taraudé correspondant 12 de la platine 5 du support supérieur 2 et dont la tête 13 s'appuie contre un épaulement annulaire 14 défini par un alésage traversant 15 de la platine 6 du support inférieur 3. La tête 13 des vis 10 et les épaulements 14 constituent des butées qui limitent de manière fiable l'écartement des supports 2, 3 et empêchent ainsi ces derniers de se séparer pendant les mouvements de flexion du patient Les organes élastiques 4 sont ainsi maintenus en permanence bien en place autour des tiges de guidage 4.

De préférence, les tiges de guidage 10 sont vissées de manière à rapprocher suffisamment les supports 2, 3 pour que les organes élastiques 4 soient dans un état de précontrainte avant la mise en place de l'implant. Une telle précontrainte permet à l'implant de supporter des charges jusqu'à un certain seuil sans compression supplémentaire des organes élastiques 4. Il est notamment possible de choisir l'état de précontrainte de telle sorte qu'une fois l'implant mis en place, il soit capable de supporter les charges exercées par le rachis, lorsque le patient est dans la station érigée, sans compression supplémentaire des organes élastiques 4. Ceci permet de garantir un maintien efficace de l'écartement des apophyses épineuses.

L'implant 1 comprend en outre, de préférence, deux plots 16 qui font saillie en regard l'un de l'autre sur les faces intérieures respectives des platines 5, 6, dans la partie centrale de l'implant 1, entre les organes élastiques 4. Ces plots 16 peuvent venir en butée l'un contre l'autre pendant des mouvements d'extension du patient pour limiter la compression des organes élastiques 4 et protéger ainsi ces derniers.

Comme on peut le voir à la figure 2, l'espace entre les organes élastiques 4 peut être utilisé pour rapprocher l'organe de fixation 7, 8 du support supérieur 2 de l'organe de fixation 7, 8 du support inférieur 3. En effet, un inconvénient des implants inter-épineux traditionnels est qu'ils nécessitent de réséquer une partie des apophyses épineuses pour dégager un espace suffisant permettant l'insertion de l'implant. Une telle résection, outre son caractère fastidieux, peut entraîner une fragilité puis une rupture des apophyses épineuses. Avec l'implant selon l'invention, une telle résection n'est pas nécessaire, ou peut être effectuée de manière minime, grâce aux évidements 7 de réception des apophyses épineuses qui sont pratiqués dans les platines 5, 6 et qui sont proches l'un de l'autre. De préférence, la distance d entre les fonds respectifs des évidements 7 est sensiblement égale voire inférieure à la hauteur h des organes élastiques 4. Ainsi, l'implant peut être mis en place sans résection des apophyses épineuses tout en comportant des organes élastiques 4 de grande hauteur et de grande capacité d'amortissement.

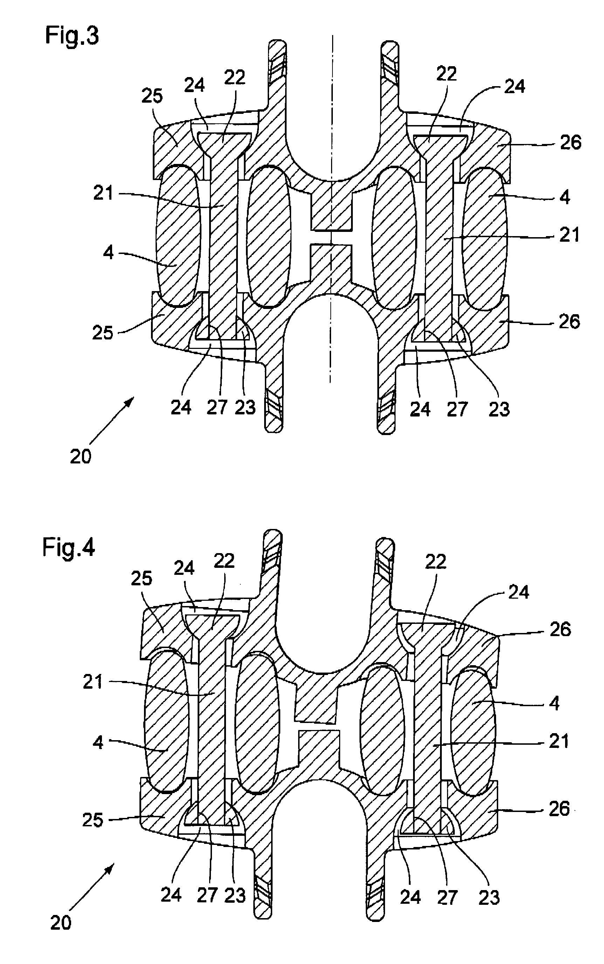

Les figures 3 et 4 montrent un implant vertébral inter-épineux 20 selon un second mode de réalisation de l'invention. Cet implant 20 diffère de l'implant 1 selon le premier mode de réalisation en ce que les tiges de guidage 21 ne sont pas vissées dans l'une des platines mais comportent à chacune de leurs extrémités une tête de forme hémisphérique 22, 23. Ces têtes hémisphériques 22, 23 reposent dans des logements de forme hémisphérique 24 mais de plus grand rayon pratiqués dans les platines 25 des supports 26. Les deux têtes 22, 23 de chaque tige de guidage 21 jouent le rôle de butées pour limiter l'écartement des supports 26. L'une au moins de ces têtes 22, 23 est un écrou 23 qui coopère avec un filetage 27 de la tige 21 pour régler l'écartement des supports 26 et donc la précontrainte des organes élastiques 4. La forme sphérique des têtes 22, 23 et des logements 24 permet aux supports 26 de s'incliner l'un par rapport à l'autre dans le plan frontal pendant un mouvement de flexion latérale du patient, comme montré à la figure 4.

La figure 5 montre comment l'implant 1 ou 20 selon l'invention peut être mis en place. L'implant est placé entre deux apophyses épineuses consécutives 30, 31 au besoin en comprimant les organes élastiques 4 pour faciliter l'insertion de l'implant puis en relâchant l'effort de compression une fois l'implant en place. Les ailettes 8 comprennent chacune une fente 32 (cf. figure 1) s'étendant dans la direction antéro-postérieure. Ces fentes 32 permettent le passage d'un ou plusieurs ligaments synthétiques servant à maintenir de manière souple les apophyses épineuses 30, 31 dans les organes de fixation 7, 8. En fonction de l'état de précontrainte des organes élastiques 4, ces ligaments synthétiques sont plus ou moins sollicités pendant des mouvements de flexion du patient. Par leur élasticité, ces ligaments synthétiques autorisent un écartement des vertèbres même lorsque l'écartement des supports 2, 3, respectivement 26, est bloqué par les butées 13, 14, respectivement 22, 23, 24. Cet écartement des vertèbres reste toutefois dans des limites physiologiques.

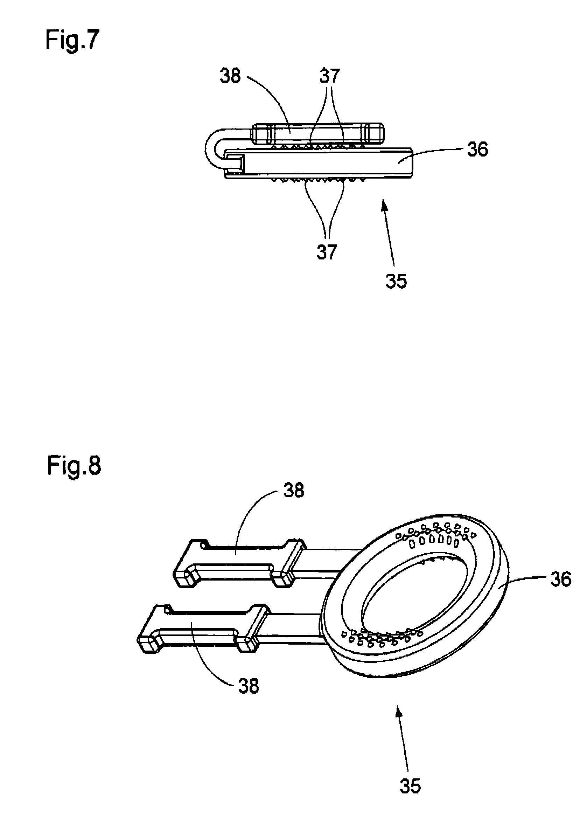

Traditionnellement, de tels ligaments synthétiques sont arrêtés et unis les uns aux autres au moyen de noeuds. Selon une caractéristique de l'invention, une pièce 35 est utilisée pour arrêter et unir les parties d'extrémité d'un ligament sans faire de noeud. Cette pièce est représentée aux figures 6 et 7. Elle comprend une partie annulaire 36 comportant des picots 37 sur ses surfaces supérieure et inférieure et deux languettes parallèles 38 rattachées à l'une de leurs extrémités à la surface périphérique externe de la partie annulaire 36 et ramenées au-dessus de la partie annulaire 36 par un pliage de 180°. La figure 8 montre la pièce d'arrêt 35 avant le pliage des languettes 38.

Comme représenté à la figure 5, la pièce d'arrêt 35 est placée libre à côté de l'implant avec sa face inférieure tournée vers l'implant. Un ligament synthétique 39 fait le tour de l'implant sensiblement dans le plan frontal en passant par les fentes 32 des ailettes 8 et en contournant l'apophyse épineuse supérieure 30 par son extrémité supérieure et l'apophyse épineuse inférieure 31 par son extrémité inférieure. Les deux bouts du ligament 39 passent dans la partie centrale de l'ouverture 40 de la partie annulaire 36 par la face inférieure de cette dernière, puis forment une boucle autour des languettes 38 respectivement pour retraverser l'ouverture 40 dans la partie périphérique de celle-ci. Les extrémités du ligament 39 sont laissées libres. En tirant sur ces extrémités, le chirurgien serre le ligament 39 dans la pièce d'arrêt 35. Les picots 37 coopèrent alors avec le ligament 39 pour assurer un bon maintien de ce dernier.

## Revendications

1. Implant vertébral inter-épineux comprenant :
- des premier et second supports (2, 3) définissant dans leur partie centrale des organes respectifs (7, 8) de fixation à des apophyses épineuses de deux vertèbres respectives, ces organes de fixation (7, 8) étant situés dans un même plan sagittal (P),
- des premier et second organes élastiquement compressibles (4) dans la direction du rachis, ces organes élastiquement compressibles (4) étant disposés entre les premier et second supports (2, 3) et de part et d'autre dudit plan sagittal (P),
**caractérisé en ce qu'**il comprend en outre des premier et second organes rigides de guidage (10) autour desquels sont disposés respectivement les premier et second organes élastiquement compressibles (4), chacun de ces organes de guidage (10) étant connecté à ses deux extrémités aux supports (2, 3) et comportant à au moins l'une de ses extrémités une butée (13) conçue pour coopérer avec une butée (14) du support correspondant (3) pour limiter l'écartement des supports (2, 3) suivant la direction du rachis.

2. Implant selon la revendication 1, **caractérisé en ce que** les butées (13) des organes de guidage (10) maintiennent les organes élastiquement compressibles (4) dans un état précontraint.

3. Implant selon la revendication 1 ou 2, **caractérisé en ce que** les organes de guidage (10) sont vissés à l'une de leurs extrémités (11) dans l'un (2) des supports (2, 3) et comportent une butée (13) à l'autre extrémité.

4. Implant selon la revendication 1 ou 2, **caractérisé en ce que** l'une au moins des extrémités (22, 23) de chaque organe de guidage (21) a une forme sphérique permettant une inclinaison des supports (26) l'un par rapport à l'autre.

5. Implant selon la revendication 4, **caractérisé en ce que** l'extrémité de forme sphérique ou l'une au moins des extrémités de forme sphérique de chaque organe de guidage (21) comprend un écrou (23) de forme sphérique.

6. Implant selon l'une quelconque des revendications 1 à 5, **caractérisé en ce que** les supports (2, 3) définissent en outre des butées respectives (16) situées entre les organes élastiquement compressibles (4) et pouvant venir en contact l'un avec l'autre pour limiter la compression des organes élastiquement compressibles (4).

7. Implant selon l'une quelconque des revendications 1 à 6, **caractérisé en ce que** les organes élastiquement compressibles (4) sont en un matériau viscoélastique.

8. Implant selon l'une quelconque des revendications 1 à 7, **caractérisé en ce que** les organes de fixation (7, 8) définissent chacun un logement (7) destiné à recevoir une apophyse épineuse, et **en ce que** la distance (d) entre les fonds respectifs des logements (7) est au plus sensiblement égale à la hauteur (h) des organes élastiquement compressibles (4).

9. Implant selon la revendication 8, **caractérisé en ce que** ladite distance (d) est inférieure à ladite hauteur (h).

10. Implant selon l'une quelconque des revendications 1 à 9, **caractérisé en ce que** les organes de fixation (7, 8) comprennent chacun des ailettes (8) entre lesquelles peut être reçue une apophyse épineuse, ces ailettes (8) comprenant chacune une ouverture (32) permettant le passage d'un ligament.

11. Dispositif comprenant un implant selon l'une quelconque des revendications 1 à 10 et une pièce d'arrêt (35) destinée à rester libre à proximité de l'implant et adaptée pour arrêter sans noeud des parties d'extrémité d'un ligament (39) de maintien de l'implant entre les apophyses épineuses.

12. Dispositif selon la revendication 11, **caractérisé en ce que** la pièce d'arrêt (35) comprend une partie annulaire (36) et des languettes parallèles (38) repliées par-dessus la partie annulaire (36).
